# EUROPEAN PATENT SPECIFICATION

(11) **EP 3 397 656 B1**
(45) Date of publication and mention of the grant of the patent: **12.02.2020**
(21) Application number: 16819354.8
(22) Date of filing: 30.11.2016
(51) Int. Cl.: C08F 2/44, C08F 226/10, A61L 27/50, A61L 27/16

(54) **ELECTRICALLY ACTIVE HYDROPHILIC BIO-POLYMERS**
ELEKTRISCH AKTIVE HYDROPHILE BIOPOLYMERE
BIOPOLYMÈRES HYDROPHILES ÉLECTRIQUEMENT ACTIFS

(30) Priority: 30.12.2015 GB 201523102
(43) Date of publication of application: 07.11.2018
(73) Proprietor: Superdielectrics Ltd, Royston, Hertfordshire SG8 5NJ (GB)
(72) Inventor: HIGHGATE, Donald James, Royston Hertfordshire SG8 5NJ (GB)
(74) Representative: Gill Jennings & Every LLP
(86) International application number: PCT/GB2016/053751
(87) International publication number: WO 2017/115064

(56) References cited:
- US-A- 4 668 506
- US-A1- 2009 232 871
- GUIMARD ET AL: "Conducting polymers in biomedical engineering", PROGRESS IN POLYMER SCIENCE, PERGAMON PRESS, OXFORD, GB, vol. 32, no. 8-9, 7 August 2007 (2007-08-07), pages 876-921, XP022188826, ISSN: 0079-6700, DOI: 10.1016/J.PROGPOLYMSCI.2007.05.012

## Description

### Field of the Invention

The present invention relates to electronically active hydrophilic polymers and their production.

### Background of the invention

Electrochemical biomedical devices such as interfaces between nervous tissue and electronic systems in prosthetic devices depend upon the transmission and control of both ions and electrons. As such, it is desirable to be able to separately control the properties of ionically conducting materials, and electronically conducting materials, in the context of biomedical applications.

Ionic conducting polymers (ICP) are materials in which the conduction process is principally dependent on ion transfer. Conventional solid ICP are typified by Nafion®, a fluorocarbon-based cationic (proton) conductor which has become the industry standard material for the production of solid polymer fuel cells and electrolysers.

Electronic conducting polymers (ECP) are well known, and are understood to mean materials in which the conduction process is principally dependent upon electron transfer. ECP include polyacetylene which has achieved electrical conductivities of 10⁷ S/m approximating to that of common metals, while commercial materials supplied as dispersions in water, e.g. polyethylenedioxythiophene:polystyrene suphonate (PEDOT:PSS, commercially available as Clevios 500®), have a conductivity of 3x10⁴ S/m and exceed the conductivity of graphite commonly used as a conductor in fuel cells. Although these materials have beneficial electronic conductive properties, their degrees of bio-acceptability have not been proven, and so have limited applicability in the bio-medical field.

GB2479449 discloses a membrane material comprising an electronically conducting polymer (ECP) and an ionically conducting polymer (ICP) with an interpenetrating region at their junction. The membrane material consists of a region of an electronically conducting polymer and a region of a hydrophilic ionically conducting polymer. The material is principally electronically conductive only in the region of the electronically conductive polymer, and any hydrophilicity is principally limited to the hydrophilic ionically conducting region of the polymer. Also in the prior art is US 4 668 506, which concerns a sustained-release polymeric hydrogel dosage form, US 2009/232871, which concerns biocompatible polymers, and Guimard et al "Conducting polymers in biomedical engineering" (progress in polymer science, vol 32, no 8-9, pages 876-921) which concerns conducting polymers with biomedical applications.

### Summary of the invention

It has been found that the polymerisation of a co-monomer solution comprising at least one hydrophobic monomer, at least one hydrophilic monomer, water, at least one amino acid, and at least one cross-linker results in the production of a new electronically active hydrophilic co-polymer. This material is homogenous and isotropic in its conductive properties, and in its water properties. It is hydrophilic, and electronically conductive, throughout its entire structure. The resulting co-polymer materials are suitable for biomedical applications by virtue of their bio-acceptability, hydrophilic properties, flexibility, and resistance to chemical degradation.

In a first aspect, the present invention provides a process of forming an electronically active hydrophilic co-polymer comprising the steps of:
providing a co-monomer solution comprising at least one hydrophobic monomer, at least one hydrophilic monomer, water, at least one amino acid and at least one cross-linker; and
polymerising the co-monomer solution;
wherein the at least one hydrophilic monomer is selected from methacrylic acid, hydroxyethyl methacrylate, ethyl acrylate, vinyl pyrrolidone, propenoic acid methyl ester, monomethacryloyloxyethyl phthalate, ammonium sulphatoethyl methacrylate, poly vinyl alcohol or a combination thereof; and
wherein the at least one hydrophobic monomer is selected from methyl methacrylate, allyl methacrylate, acrylonitrile, methacryloxypropyl-tris(trimethylsiloxy)silane, 2,2,2-trifluoroethyl methacrylate, or a combination thereof.

In a second aspect, the present invention provides a homogenous, isotropic electronically active hydrophilic co-polymer obtainable by the process according to the first aspect of the invention.

In a third aspect, the present invention provides a co-monomer solution, as defined above, comprising, at least one hydrophobic monomer, at least one hydrophilic monomer, water, at least one amino acid, and at least one cross-linker.

Further aspects are defined in the independent claims and include a variety of biocompatible medical devices. One such biocompatible medical device is one that comprises a supercapacitor. As a result of their improved electronic properties, the co-polymers described herein may be used as the electrolyte component within the supercapacitor system. When the co-polymers described herein are used in this context, the resulting supercapacitor achieves particularly high capacitance values, allowing improved performance of the medical device in question. Furthermore, as a result of the mechanical properties of the co-polymers described herein are such that the resulting supercapacitor does not require an additional separator.

### Description of the preferred embodiments

As used herein, the term "monomer" takes its usual definition in the art, and so refers to a molecular compound that may chemically bind to another monomer to form a polymer.

As used herein, the term "co-monomer solution", takes its usual definition in the art, and so refers to a solution of miscible monomers that, when polymerised, forms a co-polymer.

As used herein, the term "cross-linker" refers to molecular compound capable of forming chemical bonds between polymer chains, and includes compounds such as methylenebisacrylamide, N-(1-Hydroxy-2,2-dimethoxyethyl)acrylamide, allyl methacrylate and ethylene glycol dimethacrylate. Allyl methacrylate and ethylene glycol dimethacrylate are preferred. The cross-linker may be hydrophobic or hydrophilic.

As used herein, the term "polymerisation initiator" takes its usual definition in the art, and so refers to an agent capable of initiating the process of chemical polymerisation, for example free-radical polymerisation. Azobisisobutyronitrile (AIBN) and-hydroxy-2-methylpriophenone are examples of such initiators. Azobisisobutyronitrile (AIBN) has utility when polymerisation is by thermal means, and 2-hydroxy-2-methylpriophenone is suitable for use with UV polymerisation.

As used herein, the term "intermediate solution" refers to a solution to which further components are added. For instance, in the context of forming the co-monomer solution, the term "intermediate solution" refers to a solution including some, but not all the components of the complete co-monomer solution.

As used herein, the term "co-polymer" takes its usual definition in the art, and so refers to a polymer whose polymer chains comprise two or more different types of monomers.

As used herein, the term "water properties" when used in relation to a polymer material, refers the properties and behaviour of that polymer material in relation to water and other aqueous environments, such as saline solution i.e. its hydrophilicity and stability in an aqueous environment.

As used herein, the term "homogenous", when used in relation to a polymer material, refers to a polymer material whose physical properties (e.g. conductive properties and water properties) are substantially uniform throughout its entire structure, i.e. they are in a single "phase".

As used herein, the term "isotropic", when used in relation to a polymer material, refers to a polymer material whose properties are the same in all orientations.

As used herein, the term "homogenous" when used in relation to a co-monomer solution, refers to a co-monomer solution comprising miscible monomers that are uniformly mixed.

As used herein, the term "hydrophilic polymer" refers to a polymer that dissolves in water when it is not cross-linked and absorbs water and swells to form a stable elastic solid when cross-linked.

As used herein, the term "hydrophilic monomer" takes its usual definition in the art, and so refers to a monomer with an affinity for water molecules. The term "hydrophobic monomer" also takes its usual definition in the art, and so refers to a monomer that repels water molecules.

As used herein, the term "amino acid" takes its usual definition in the art, and so refers to an organic compound with amino and carboxylic acid functional groups, and a side-chain that is specific to each amino acid. The term encompasses the traditional "natural" amino acids but also any compound with an amino acid backbone (i.e. with any side-chain).

As used herein, the term "electrically active" takes its usual definition in the art, and so can encompass both electronically active and ionically active materials.

As used herein, the term "electronically active material" takes its usual definition in the art, and refers to a material in which the conduction process is principally dependent upon electron transfer, or in which an electron is yielded as an output at an interface.

As used herein, the term "ionically active material" takes its usual definition in the art, and refers to a material in which the conduction process is principally dependent on ion transfer.

As used herein, the terms "biocompatible" and "bio-acceptable" are used interchangeably throughout, and take their usual definition in the art, that is, the ability of a particular material to be in contact with a living system without producing an adverse effect.

As used herein, the term "liquid electrolyte" takes its usual definition in the art, and so refers to a solution of cations (such as potassium, sodium, calcium and magnesium) and anions (such as chloride, carbonate and phosphate) dissolved in a solvent, such as water, acetonitrile, propylene carbonate or tetrahydrofuran. As used herein, the term "aqueous electrolyte" takes its usual definition in the art, and so refers to an aqueous solution containing cations (such as potassium, sodium, calcium and magnesium) and anions (such as chloride, carbonate and phosphate).

In a first aspect the present invention provides a process of forming an electronically active hydrophilic co-polymer comprising the steps of:
providing a co-monomer solution comprising at least one hydrophobic monomer, at least one hydrophilic monomer, water, at least one amino acid and at least one cross-linker, and
polymerising the co-monomer solution;
wherein the at least one hydrophilic monomer is selected from methacrylic acid, hydroxyethyl methacrylate, ethyl acrylate, vinyl pyrrolidone, propenoic acid methyl ester, monomethacryloyloxyethyl phthalate, ammonium sulphatoethyl methacrylate, poly vinyl alcohol or a combination thereof; and
wherein the at least one hydrophobic monomer is selected from methyl methacrylate, allyl methacrylate, acrylonitrile, methacryloxypropyl-tris(trimethylsiloxy)silane, 2,2,2-trifluoroethyl methacrylate, or a combination thereof.

Preferably, the at least one amino acid is selected from phenylalanine, tryptophan, histidine, tyrosine and ethylenediaminetetraacetic acid (EDTA), or a combination thereof. It was found that using these amino acids results in a hydrophilic polymer with particularly good electronic properties. Without wishing to be bound by theory, it is thought that the electron conjugation within the aromatic system or the delocalised electron lone pairs favourably alters the electronic properties of the polymer material. Preferably, the co-monomer solution comprises one amino acid or two different amino acids.

Preferably, the amino acid is selected from a natural amino acid. More preferably, the amino acid is selected from an amino acid (preferably a natural amino acid) comprising, in its side chain, an aromatic group. More preferably, the at least one amino acid is selected from phenylalanine, tryptophan, histidine and tyrosine or a combination thereof. Still more preferably, the at least one amino acid is selected from phenylalanine and tryptophan, or a combination thereof. In some embodiments, the co-monomer solution comprises phenylalanine and tryptophan.

In a preferred embodiment, the amino acid is added in the form of a solid to the existing components of the co-monomer solution. The solid amino acid may be in the form of a powder.

The at least one hydrophilic monomer is selected from methacrylic acid, hydroxyethyl methacrylate (e.g. 2-hydroxyethyl methacrylate), ethyl acrylate, vinyl pyrrolidone (e.g. n-vinyl-2-pyrrolidone), propenoic acid methyl ester (e.g. propenoic acid 2-methyl ester), monomethacryloyloxyethyl phthalate, poly-vinyl alcohol, ammonium sulphatoethyl methacrylate, or a combination thereof. Preferably, the co-monomer solution comprises one hydrophilic monomer.

Preferably, the at least one hydrophilic monomer is selected from vinyl-2-pyrrolidone and 2-hydroxyethyl methacrylate, or a combination thereof. More preferably, the at least one hydrophilic monomer is selected from 1-vinyl-2-pyrrolidone (VP) and 2-hydroxyethyl methacrylate, or a combination thereof.

The at least one hydrophobic monomer is selected from methyl methacrylate, acrylonitrile, methacryloxypropyltris(trimethylsiloxy)silane, 2,2,2-trifluoroethyl methacrylate, allyl methacrylate, or a combination thereof. Preferably, the co-monomer solution comprises one hydrophobic monomer.

Preferably, the at least one hydrophobic monomer is selected from acrylonitrile and methyl methacrylate, or a combination thereof.

Preferably, the at least one cross-linker is selected from allyl methacrylate or ethylene glycol dimethacrylate. Preferably, the co-monomer solution comprises one hydrophilic monomer.

It will be appreciated from the definitions above, that the terms used above are not necessarily mutually exclusive. For example, the terms "hydrophobic monomer" and "cross-linker" are not necessarily mutually exclusive. In the present invention, the hydrophobic monomer and the cross-linker may be the same or different.

The hydrophobic monomer may, in certain embodiments, be the same as the cross-linker. For example, in certain embodiments, both the cross-linker and the hydrophobic monomer may be allyl methacrylate.

In some embodiments, the hydrophilic monomer and/or the hydrophobic monomer are non-cross-linking. There is no overlap between the terms "non-cross-linking hydrophobic monomer", "non-cross-linking hydrophilic monomer" and "cross-linker". In these embodiments, the cross-linker, the hydrophobic monomer and the hydrophilic monomers are different chemical species.

Preferably, the polymerisation step is carried out by thermal, UV or gamma radiation.

More preferably, the polymerisation step is carried out by UV or gamma radiation.

In a preferred embodiment, the co-monomer solution further comprises a polymerisation initiator. The polymerisation initiator may be azobisisobutyronitrile (AIBN) or 2-hydroxy-2-methylpriophenone.

The presence of a polymerisation initiator is particularly preferred when the polymerisation is by thermal or UV radiation. In one embodiment, the polymerisation is by thermal means and the initiator is azobisisobutyronitrile (AIBN). In another embodiment, the polymerisation is by UV radiation and the initiator is 2-hydroxy-2-methylpriophenone.

The individual components of the co-monomer solution should be included in sufficient quantities such that they mix uniformly thereby forming a homogenous solution. The hydrophobic monomer may be present in an amount of 5% to 80% by weight, preferably, 5% to 60% by weight, most preferably 40-60% by weight based on the total weight of the co-monomer solution. The hydrophilic monomer may be present in an amount of 5% to 90% by weight, preferably 5% to 80% by weight, most preferably 40-60% by weight based on the total weight of the co-monomer solution. The cross-linker agent may be present in the co-monomer solution in an amount of 1% to 25% by weight, preferably 2% to 15% by weight, most preferably 2% to 10% by weight based on the total weight of the co-monomer solution. The amino acid may be present in an amount of 0.05% to 30% by weight, preferably 0.1% to 10% by weight, most preferably 0.5% to 1.5% by weight based on the total weight of the co-monomer solution.

The amount of water in the co-monomer solution must be sufficient to provide a uniformly mixed homogenous solution, and must be sufficient to dissolve the at least one amino acid, which is insoluble in the monomer components and the cross-linker agent. The amount of water in the co-monomer solution may be 1% to 50% by weight, preferably 5% to 50% by weight, most preferably 5% to 15% by weight based on the total weight of the co-monomer solution.

In a preferred embodiment, the co-polymer is hydrated following polymerisation. This hydration step may be carried out using distilled deionized (DD) water, or an aqueous solution, such as saline. When saline solution is used for the hydration step, the saline solution preferably has 0.002 g/cc to 0.1 g/cc of NaCl in water, more preferably 0.009 g/cc of NaCl in water. It is preferred that this hydration step results in the amount of water in the co-polymer being at least 10% by weight, preferably at least 20% by weight, based on the total weight of the hydrated co-polymer. Without wishing to be bound by theory, when water is present in this quantity, then it can act as a "plasticizer" and enable the other components of the co-polymer to have sufficient intermolecular mobility such that the conformation of the co-monomer self-organises over time. For example, this self-organisation can occur within a period of about 7-14 days. It has been observed that, following manufacture and/or further hydration, the electrical properties of the co-polymer improve over time.

The co-monomer solution may be provided and polymerised using UV, gamma or thermal radiation. The UV or gamma radiation may be carried out under ambient temperature and pressure, whilst thermal polymerisation may be carried out at temperatures up to 70°C.

The co-monomer solution may be provided by mixing the solution components in a number of different orders. In one embodiment, the hydrophilic and hydrophobic monomers are mixed first, and then the water is added, followed by the addition of the amino acid and the cross-linker. In another embodiment, the amino acid may be dissolved in water, and the resultant amino acid solution added to a mixture of the hydrophilic monomers, hydrophobic monomers and the cross-linker.

Preferably the co-monomer solution is provided by:
mixing the at least one hydrophobic monomer and the at least one hydrophilic monomer in water to form an intermediate solution; and
adding the at least one amino acid and the cross-linker to the intermediate solution to form the co-monomer solution;
wherein the hydrophobic monomer and the cross-linker are different chemical species.

This is a particularly preferred embodiment of the first aspect of the invention, as surprisingly, it was found that mixing the polymer components in this manner enabled up to twice as much amino acid to dissolve in the co-monomer solution as would otherwise dissolve in the amount of water present in the co-monomer solution. Dissolving more amino acid in the co-monomer solution is desirable as it results in more amino acid being incorporated in the polymer, and so provides particularly improved electronic properties. This is demonstrated in the Examples.

Preferably, the ratio, by volume, of hydrophilic monomer and hydrophobic monomer : water, in the intermediate solution, is from 2:1 to 10:1. More preferably, the ratio, by volume, of hydrophilic monomer and hydrophobic monomer: water, in the intermediate solution, is 4:1.

Preferably, the ratio, by volume, of hydrophilic monomer : hydrophobic monomer, in the intermediate solution, is from 10:1 to 1:5. More preferably, the ratio, by volume, of hydrophilic monomer : hydrophobic monomer, in the intermediate solution, is 1:1.

Most preferably, the ratio, by volume, of hydrophilic monomer : hydrophobic monomer: water in the intermediate solution is 1 : 1 : 0.5.

Preferably, the co-polymer is hydrated after polymerisation. More preferably, the co-polymer is hydrated such that the hydrated co-polymer comprises at least 10 wt% water, preferably at least 20 wt% water, based on the total weight of the hydrated co-polymer.

Preferably, the co-polymer is stored for at least 7 days, preferably for at least 14 days, following hydration.

In a preferred embodiment, a process of forming an electronically active hydrophilic co-polymer comprises the steps of:
providing a co-monomer solution consisting of a hydrophobic monomer, a hydrophilic monomer, water, an amino acid and a cross-linker; and
polymerising the co-monomer solution.

In a second aspect the present invention provides a homogenous and isotropic electronically active hydrophilic co-polymer obtainable by the process according to any of the embodiments set out with respect to the first aspect of the invention. It is believed that such a homogeneous co-polymer is novel.

In a third aspect, the present invention provides a co-monomer solution comprising at least one hydrophobic monomer, at least one hydrophilic monomer, water, at least one amino acid and at least one cross-linker.

Preferred hydrophobic monomers, hydrophilic monomers, amino acids and cross-linkers are defined above.

The above-mentioned co-monomer solutions result in the homogenous, isotropic electronically active hydrophilic co-polymers according to the present application.

Co-polymers of the invention can be used in a variety of applications, and are particularly useful where a biocompatible material is desired. For example, the co-polymers of the invention may be used in biocompatible medical devices, such as nerve contact interfaces, cochlear implants and pacemakers. The hydrophilic properties of the materials of the inventions make them particularly suited in biomedical applications.

In one embodiment, the biocompatible medical device in question includes a supercapacitor, where the co-polymers disclosed herein are used as the electrolyte component within the supercapacitor system. As will be appreciated by the skilled person, supercapacitors generally comprise two electrodes and an electrolyte component located therebetween. The maximum capacitance value achieved by a supercapacitor may depend on the nature of the electrolyte as well as the nature of the electrodes. As will also be appreciated by the skilled person, there are multiple different kinds of supercapacitor systems. These include double-layer supercapacitors, pseudo-capacitive supercapacitors, and hybrid supercapacitors. Double-layer supercapacitors typically comprise carbon electrodes that are of comparatively low cost. The capacitance of double-layer supercapacitors is largely electrostatic capacitance. Meanwhile, pseudo-capacitive supercapacitors comprise comparatively higher cost electrodes that are capable of undergoing an oxidation-reduction (redox) reaction together with the electrolyte. Such redox active electrodes can comprise, for example, lanthanum ruthenium or vanadium. The capacitance of pseudo-capacitive supercapacitors is therefore significantly increased (or augmented) by electrochemical capacitance. Hybrid supercapacitors comprise a combination of electrodes with differing characteristics, and can for example comprise one carbon electrode and one electrode capable of undergoing a redox reaction with the electrolyte. The capacitance of hybrid supercapacitors is therefore a combination of electrostatic capacitance and electrochemical capacitance. Conventionally, the electrolyte component within the above supercapacitor systems is a liquid electrolyte, and such liquid electrolytes are typically not bio-acceptable and cannot therefore be used in vivo without significant risk and extensive sealing systems

When the co-polymers disclosed herein are used in place of a conventional liquid electrolyte of a supercapacitor, the resulting supercapacitor achieves particularly high capacitance values. When the co-polymers disclosed herein are used in a double-layer supercapacitor, the capacitance values achieved are three orders of magnitude larger relative to the capacitance values that are achieved with a conventional liquid electrolyte. When the co-polymers disclosed herein are used in a pseudo-capacitive supercapacitor, these high capacitance values are maintained. Good capacitance values are also achieved in the context of hybrid supercapacitors. In summary, for a given supercapacitor system and with a given electrode, the maximum capacitance is increased when using the co-polymers disclosed herein as the electrolyte component within a supercapacitor. Furthermore, the electrolytic properties of these co-polymers remain excellent when hydrated in medical grade saline solution, thereby providing electrical storage devices particularly suitable for medical applications. Further, the co-polymers remain stable across a commercially acceptable voltage range.

Furthermore, the co-polymers disclosed herein are mechanically stable. As a result of these improved mechanical properties, a supercapacitor including the co-polymers disclosed herein as the electrolyte component does not require an additional separator. Conventionally, when a liquid electrolyte is used within a supercapacitor system, it is necessary for the supercapacitor to further comprise an additional separator in order to maintain separation between the two electrodes. When the co-polymers described herein are used in place of the conventional liquid electrolyte, their mechanical properties and self-supporting nature is such that separation between the electrodes is maintained even in absence of an additional separator.

In another embodiment, the co-polymers disclosed herein are used in a biocompatible sensing system. Sensing systems may include one or more chemical components, where these chemical components are capable of detecting a particular compound. Such sensing systems have wide applicability in a biomedical context. Advantageously, these one or more chemical components may be dispersed throughout the structure of the co-polymers disclosed herein, and the resulting co-polymer included in the sensing system. The co-polymers disclosed herein act as a support matrix for the chemical components, wherein the chemical components are stably retained within the co-polymer structure, and their sensing ability preserved. The particular compounds detected by such sensing systems can include glucose. The skilled person will be familiar with the chemical components capable of detecting glucose, and such chemical components can include Benedict's reagent (which comprises anhydrous sodium carbonate, sodium citrate and copper(II) sulfate pentahydrate).

In a further embodiment, the co-polymers disclosed herein are used in a photovoltaic cell. The optical transparency of the co-polymers disclosed herein allows efficient functioning of a photovoltaic cell.

In another embodiment, the co-polymers disclosed herein may be used to form an electrically conducting adhesive junction, wherein the adhesive junction is positioned between adjacent electrically conducting components. Preferably, the adjacent electrically conducting components together with the adhesive junction form a stack of integrated circuits, such as a stack of 2D electrical chips, that may be included in biomedical devices.

The present invention will now be demonstrated according to the following examples.

### Example 1

### Method 1 in which the amino acids or acids are dissolved in water the resulting solution being added to the other co-monomers.

A solution of phenylalanine in water was formed by dissolving 0.32 g of phenylalanine in 10 ml of water at 50 °C whilst stirring using a magnetic stirrer bar. (This was the maximum concentration possible at the given temperature and was in agreement with published data relating concentration to temperature). 25% by volume (0.5 ml) of the aqueous solution was then added drop-wise to a stirred mixture consisting of 1ml of acrylonitrile and 1ml of 1-vinyl-2-pyrrolidone (2ml total). 0.075 ml of allyl methacrylate as the cross-linker and 0.05 ml of 2-hydroxy-2-methylpriophenone as the initiator was then added to the mixture. It was then cured under UV for approximately 10 minutes until the result was a solid cross-linked co-polymer. Alternative initiators such as AIBN can be used for thermal polymerisation.

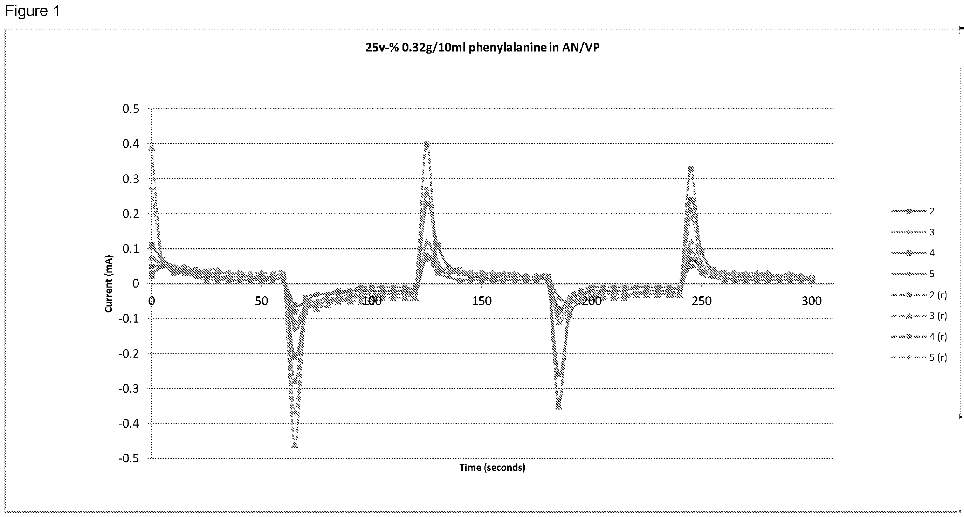

The conductivity was tested and the results are shown in Table 1 (below) and in Figure 1.

**Table 1 AN/VP (2 ml) with 25 v-% 0.32 g/10 ml phenylalanine a sample was polymerised after every heating cycle (50°C), and the sample conductivity retested (r) after 7 days.**

| No. of heating cycles | Expansion ratio | Minimum current (mA) | Maximum current (mA) |
|---|---|---|---|
| 1 | 1.11 | 0.02 | 0.18 |
| 2 | 1.09 | 0.01 | 0.08 |
| 3 | 1.17 | 0.02 | 0.12 |
| 4 | 1.25 | 0.01 | 0.20 |
| 5 | 1.2 | 0.01 | 0.13 |
| | | | |
| | | | |
| | | | |

| Samples tested after 7 days | | Minimum current (mA) | Maximum current (mA) |
|---|---|---|---|
| 1 (r) | - | - | - |
| 2 (r) | | 0.02 | 0.08 |
| 3 (r) | | 0.01 | 0.46 |
| 4 (r) | | 0.01 | 0.34 |
| 5 (r) | | 0.02 | 0.37 |

The material was tested in two ways:
(i) to establish the effect of thermal cycling because in other tests it was demonstrated that crystals of amino acids formed as the result of thermal cycling of the monomer mixture (temperatures from ambient to 50°C) and it was necessary show any effect of crystal growth on electrical properties.
   Allowing for the changing expansion ratio (implying changing water uptake after thermal cycling of the monomer mixture) no correlation with thermal cycling was apparent.
(ii) to establish the change of electrical properties with time after manufacture. It was clear that the peak current increased with time if the samples were stored at ambient temperature and hydration was maintained. In this case the tests were stopped after 7 days when an increase of between 2 and 4 times the initial current was measured.

### Example 2

### Method 2 in which the amino acid or acids are dissolved in a pre-mixed solution consisting of the other co-monomers and water.

A solution was formed by mixing 1 ml of acrylonitrile and 1 ml of 1-vinyl-2-pyrrolidone with 0.5 ml of water. 0.02 g of phenylalanine was added and the mixture was heated at 50 °C and stirred with a magnetic stirrer bar. It was found that the amount of amino acid that could be completely dissolved into the monomer+water mixture exceeded the maximum concentration possible at the given temperature possible by Method 1 and significantly exceeded the amount given in the published data.

0.075 ml of allyl methacrylate as the cross-linker and 0.05 ml of 2-hydroxy-2-methylpriophenone as the initiator was added to the mixture. It was then cured under UV for 10 minutes to produce a solid cross-linked co-polymer.

The cross-linked polymers are hydrated in DD water until equilibrium has reached and the electrical properties are tested periodically over 13 days. The hydrated samples are kept sealed so that water loss is minimised and the expansion ratio measured to determine if additional water is necessary to maintain the hydration level for the measurements.

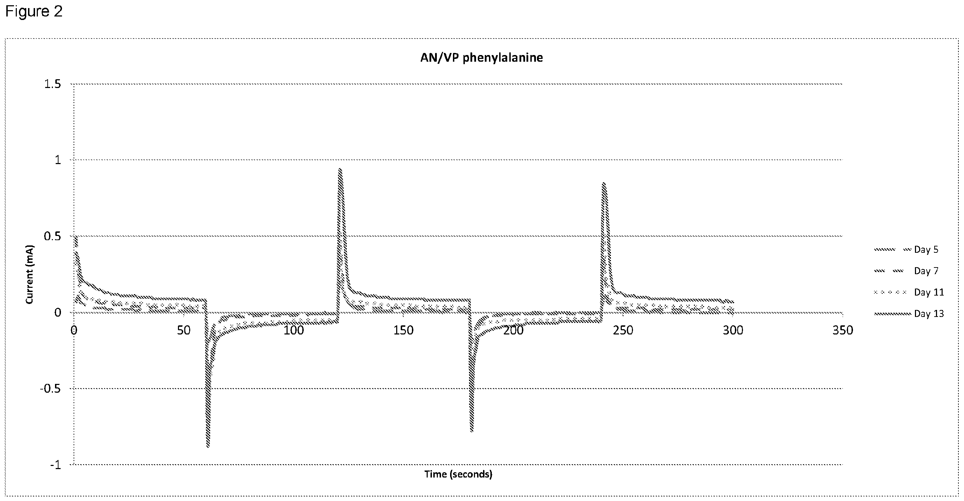

The electrical properties are shown in Figure 2 and tabulated in Table 2 (below). As can be seen the maximum current measured increased by a factor of 30 times over the test period.

**Table 2: AN/VP + H₂O with 0.02g phenylalanine**

| No. of days after maximum hydration reached | Expansion ratio | Minimum current (mA) | Maximum current (mA) |
|---|---|---|---|
| 0 | 1.14 | 0.01 | 0.03 |
| 1 | | 0.01 | 0.07 |
| 5 | | 0.01 | 0.20 |
| 7 | | 0.01 | 0.49 |
| 11 | | 0.03 | 0.83 |
| 13 | | 0.07 | 0.93 |

### Example 3

### Samples made with a mixture of two amino acids using method 2 in which the amino acids are dissolved in a pre-mixed solution consisting of the other co-monomers and water.

A solution was formed by mixing 1 ml of acrylonitrile and 1 ml of 1-vinyl-2-pyrrolidone with 0.5 ml of water. 0.02 g of phenylalanine and 0.06 g of tryptophan was added and the mixture was stirred and heating at 50 °C with a magnetic stirrer bar. 0.075 ml of allyl methacrylate as the cross-linker and 0.0 5ml of 2-hydroxy-2-methylpriophenone as the initiator was added to the mixture. It was then cured under UV to produce a cross-linked co-polymer.

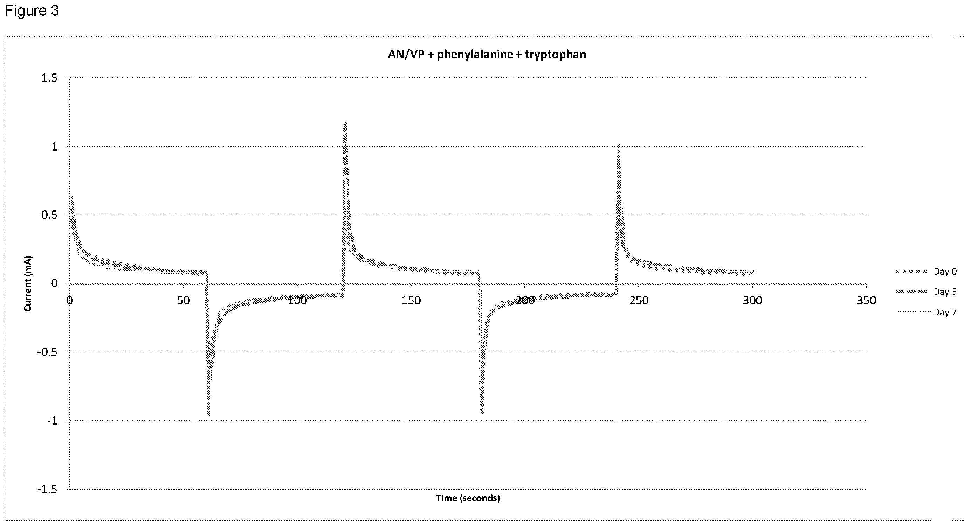

The conductivity was tested and the results are shown in Figure 3. The electrical measurements again showed an increase in maximum current with time after hydration.

### Examples 4-5

The terminology in the table below applies across examples 4 to 5:

| Acronym | Component |
|---|---|
| PHENYL | Phenylalanine |
| EDTA | Ethylenediaminetetra-acetic acid disodium dehydrate |

Across Examples 4-5, various co-polymers were prepared. The co-polymers across Examples 4-5 are listed in the table below, and were prepared using a similar methodology to that of examples 1-3: The abbreviated term listed in the "co-polymer" column of the table below will be used throughout examples 4-5:

| Co-polymer | Composition |
|---|---|
| VP:PHENYL:H₂O | 2ml VP, 0.02g Phenylalanine, 1ml H₂O, 0.15ml acrylonitrile |
| VP:EDTA:H₂O | 2ml VP, 0.04g EDTA, 1ml H₂O, 0.15ml acrylonitrile |

The electrical properties of the co-polymers of examples 4-5 were tested under potentiostatic conditions within either a cylindrical electrode device, or a flat electrode device.

The cylindrical electrode device comprises working and counter electrodes are glassy carbon rods with a cross-section of 0.06 cm². The electrode surfaces are macroscopically smooth, thus their cross-sections can be taken as the effective electroactive area. The quasi-reference electrode consists of a Ag wire inserted in the active polymer film.

The flat electrode device was used in order to assess the suitability of these materials for large geometric areas (above 5 cm²).

All measurements were recorded with an Ivium - Compactstat under ambient conditions and humidity. Irreversible changes in the co-polymer structure (e.g. over-oxidation) were monitored by systematic analysis of the open-circuit potential between the working and reference electrodes.

### Example 4

Two VP:EDTA:H₂O co-polymers were prepared using a similar methodology to that of examples 1-3. The first co-polymer was then hydrated in double distilled water, and the second co-polymer was hydrated in saline (0.9 g NaCl in 100 ml double distilled water).

Two VP:PHENYL:H₂O co-polymers were prepared using a similar methodology to that of examples 1-3. The first co-polymer was then hydrated in double distilled water, and the second co-polymer was hydrated in saline (0.9 g NaCl in 100 ml double distilled water).

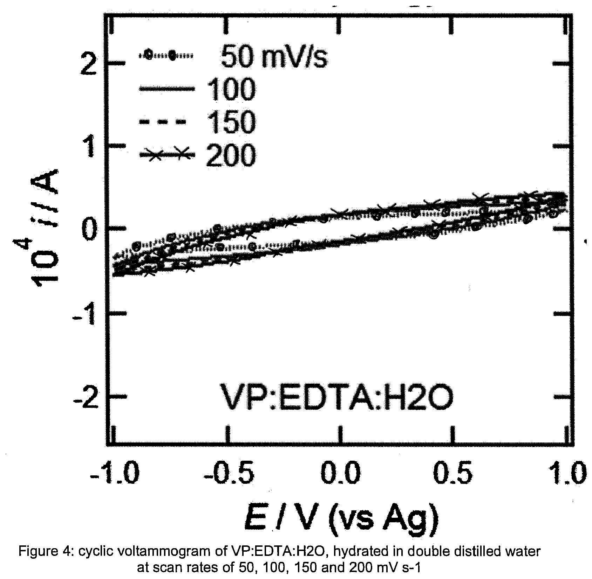

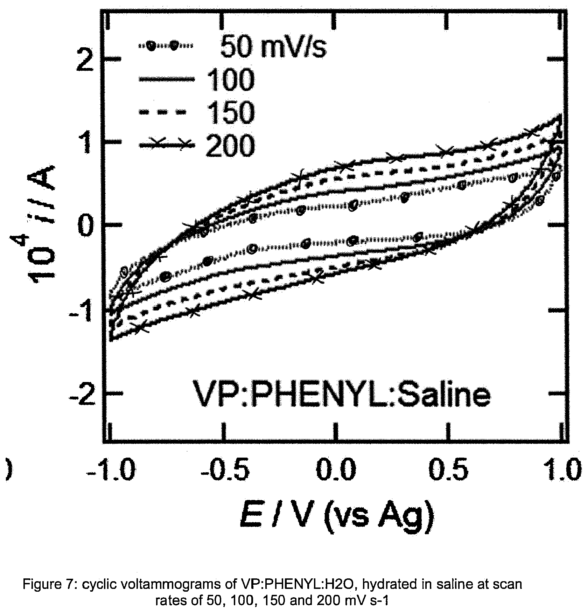

The electrical properties of each of the above four co-polymers were tested, and the results are shown in Figure 4 (VP:EDTA:H₂O, hydrated in double distilled water), Figure, 5 (VP:EDTA:H₂O, hydrated in saline), Figure, 6 (VP:PHENYL:H₂O, hydrated in double distilled water), and Figure 7 (VP:PHENYL:H₂O, hydrated in saline).

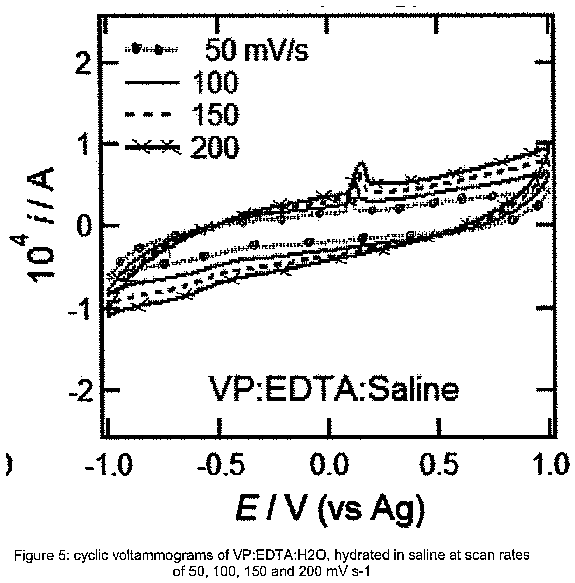

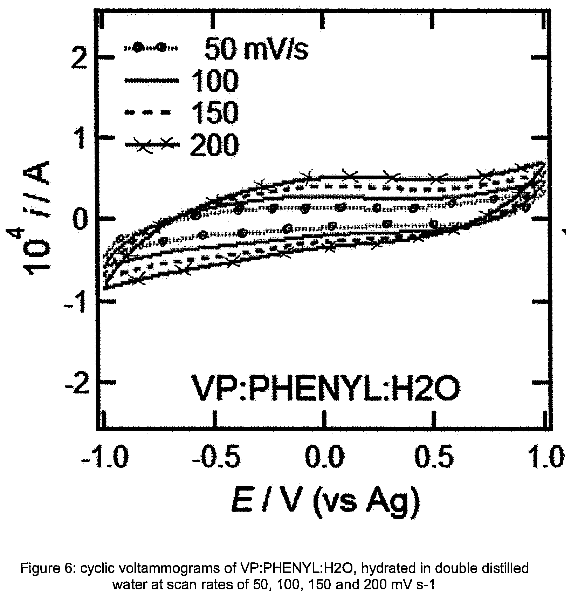

Figures 4-7 are cyclic voltammograms at scan rates of 50, 100, 150 and 200 mV s-1. These figures show an increase in capacitive current upon saline hydration at a given potential scan rate. These figures indicate that after hydration, there is ion impregnation throughout the co-polymer matrix, which enables the formation of a more compact electrochemical double layer at the electrode surface. These results therefore support the possibility of incorporating chemical components in the polymer matrix for use in sensing applications.

### Example 5

A VP:PHENYL:H₂O co-polymer was prepared using a similar methodology to that of examples 1-3. The co-polymer was then hydrated in saline solution.

A VP:EDTA:H₂O co-polymer was prepared using a similar methodology to that of examples 1-3. The co-polymer was then hydrated in saline solution.

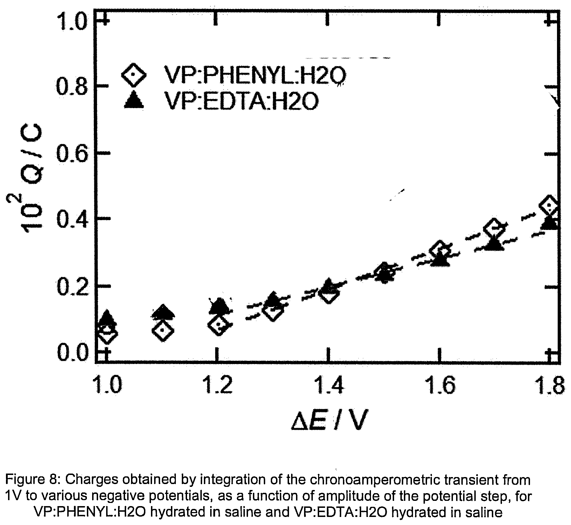

The electrical properties of each of the above two co-polymers were tested and the results shown in Figure 8. Figure 8 plots the charges obtained by integration of the chronoamperometric transient from 1V to various negative potentials, as a function of amplitude of the potential step.

The Figure shows that each co-polymer displays supercapacitive behaviour. The capacitance of each co-polymer is in the order of 0.005 F cm⁻².

### Example 6

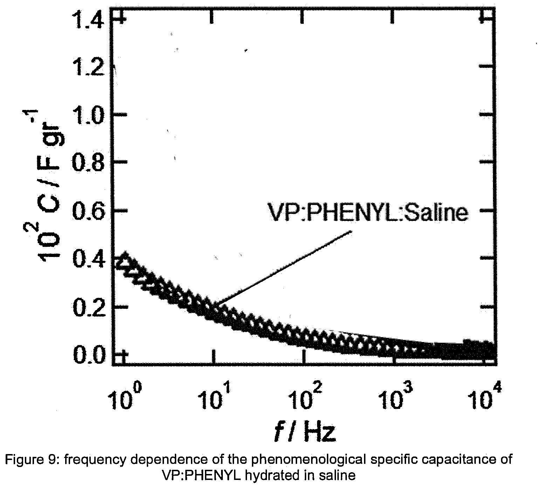

A VP:PHENYL co-polymer was prepared using a similar methodology to that of examples 1-3. The co-polymer was then hydrated in saline. The electrical properties were tested. The results are shown in Figure 9.

Figure 9 shows the frequency dependence of the phenomenological specific capacitance of the co-polymer. Capacitance values were obtained by examining the electrochemical impedance spectroscopy at the open circuit potential. The specific capacitance approaches values close to 0.004 Fg⁻¹.

## Claims

1. A process of forming a cross-linked electronically active hydrophilic co-polymer comprising the steps of:
providing a co-monomer solution comprising at least one hydrophobic monomer, at least one hydrophilic monomer, water, at least one amino acid and at least one cross-linker; and
polymerising the co-monomer solution;
wherein the at least one hydrophilic monomer is selected from methacrylic acid, hydroxyethyl methacrylate, ethyl acrylate, vinyl pyrrolidone, propenoic acid methyl ester, monomethacryloyloxyethyl phthalate, ammonium sulphatoethyl methacrylate, poly vinyl alcohol or a combination thereof; and
wherein the at least one hydrophobic monomer is selected from methyl methacrylate, allyl methacrylate, acrylonitrile, methacryloxypropyl-tris(trimethylsiloxy)silane, 2,2,2-trifluoroethyl methacrylate, or a combination thereof.

2. The process according to claim 1, wherein the at least one amino acid is selected from phenylalanine, tryptophan, histidine, ethylenediaminetetraacetic acid (EDTA) and tyrosine, or a combination thereof, preferably wherein the at least one amino acid is selected from phenylalanine and tryptophan, or a combination thereof.

3. The process according to any preceding claim, wherein the at least one hydrophilic monomer is selected from vinyl pyrrolidone and hydroxyethyl methacrylate, or a combination thereof.

4. The process according to any preceding claim, wherein the at least one hydrophobic monomer is selected from acrylonitrile and methyl methacrylate, or a combination thereof.

5. The process according to any preceding claim, wherein the crosslinking agent is allyl methacrylate or ethylene glycol dimethacrylate.

6. The process according to any preceding claim, wherein both the cross-linker and the hydrophobic monomer is allyl methacrylate.

7. The process according to any preceding claim wherein the polymerisation step is carried out by thermal, UV or gamma radiation, preferably wherein the polymerisation step is carried out by UV or gamma radiation.

8. The process according to any preceding claim, wherein the co-monomer solution further comprises a polymerisation initiator, preferably wherein the polymerisation initiator is azobisisobutyronitrile or 2-hydroxy-2-methylpriophenone.

9. The process according to any of claims 1-5 or 7-8, wherein the co-monomer solution is provided by adding the amino acid, in the form of a solid, to at least one of the remaining components of the co-monomer solution, preferably wherein the co-monomer solution is provided by:
a. mixing the at least one hydrophobic monomer and the at least one hydrophilic monomer in water to form an intermediate solution; and
b. adding the at least one amino acid and the cross-linker to the intermediate solution to form the co-monomer solution
wherein the hydrophobic monomer and the cross-linker are different chemical species.

10. The process according to any preceding claim, further comprising the step of hydrating the co-polymer after polymerisation, preferably wherein either the co-polymer is stored for at least 7 days following hydration, or wherein the co-polymer is hydrated such that the hydrated co-polymer comprises at least 10 wt% water, based on the total weight of the hydrated co-polymer.

11. A homogenous, isotropic electronically active hydrophilic co-polymer obtainable by the process according to any of claims 1-10.

12. A co-monomer solution, according to claim 1, comprising at least one hydrophobic monomer, at least one hydrophilic monomer, water, at least one amino acid and at least one cross-linker.

13. A biocompatible medical device, or a flexible biocompatible nerve contact device, or a cochlear implant, or a pacemaker electrode, comprising a co-polymer according to claim 11.

14. A biocompatible medical device comprising a supercapacitor, wherein the supercapacitor comprises two electrodes and a co-polymer according to claim 11 located therebetween, preferably wherein the device is a nerve contact device, a cochlear implant, or a pacemaker.

15. A biocompatible sensing system comprising a co-polymer according to claim 11 with a chemical component dispersed throughout its structure, wherein said chemical component is capable of detecting a particular compound.

## Patentansprüche

1. Vorgang zur Herstellung eines vernetzten, elektronisch aktiven hydrophilen Copolymers, das die folgenden Schritte umfasst:
Bereitstellen einer Co-Monomerlösung, die mindestens ein hydrophobes Monomer, mindestens ein hydrophiles Monomer, Wasser, mindestens eine Aminosäure und mindestens einen Vernetzer umfasst; und
Polymerisieren der Co-Monomerlösung;
wobei das mindestens eine hydrophile Monomer ausgewählt ist aus Methacrylsäure, Hydroxyethylmethacrylat, Ethylacrylat, Vinylpyrrolidon, Propensäuremethylester, Monomethacryloyloxyethylphthalat, Ammoniumsulfat-Ethylmethacrylat, Polyvinylalkohol oder einer Kombination davon; und
wobei das mindestens eine hydrophobe Monomer ausgewählt ist aus Methylmethacrylat, Allylmethacrylat, Acrylnitril, Methacryloxypropyl-tris(trimethylsiloxy)silan, 2,2,2-Trifluorethylmethacrylat oder einer Kombination davon.

2. Vorgang nach Anspruch 1, worin die mindestens eine Aminosäure ausgewählt ist aus Phenylalanin, Tryptophan, Histidin, Ethylendiamintetraessigsäure (EDTA) und Tyrosin oder einer Kombination davon, wobei die mindestens eine Aminosäure vorzugsweise ausgewählt ist aus Phenylalanin und Tryptophan oder einer Kombination davon.

3. Vorgang nach einem der vorhergehenden Ansprüche, wobei das mindestens eine hydrophile Monomer ausgewählt ist aus Vinylpyrrolidon und Hydroxyethylmethacrylat oder einer Kombination davon.

4. Vorgang nach einem der vorhergehenden Ansprüche, wobei das mindestens eine hydrophobe Monomer ausgewählt ist aus Acrylnitril und Methylmethacrylat oder einer Kombination davon.

5. Vorgang nach einem vorhergehenden Anspruch, wobei das Vernetzungsmittel Allylmethacrylat oder Ethylenglykol-Dimethacrylat ist.

6. Vorgang nach einem beliebigen vorhergehenden Anspruch, wobei sowohl der Vernetzer als auch das hydrophobe Monomer Allylmethacrylat ist.

7. Vorgang nach einem beliebigen vorhergehenden Anspruch, wobei der Polymerisationsschritt durch thermische, UV- oder Gammastrahlung durchgeführt wird, wobei der Polymerisationsschritt vorzugsweise durch UV- oder Gammastrahlung durchgeführt wird.

8. Vorgang nach einem beliebigen vorhergehenden Anspruch, wobei die Co-Monomerlösung ferner einen Polymerisationsinitiator umfasst, wobei der Polymerisationsinitiator vorzugsweise Azobisisobutyronitril oder 2-Hydroxy-2- methylpriophenon ist.

9. Vorgang nach einem der Ansprüche 1-5 oder 7-8, wobei die Co-Monomerlösung durch Zugabe der Aminosäure in Form eines Feststoffs zu mindestens einer der verbleibenden Komponenten der Co-Monomerlösung hergestellt wird, wobei die Co-Monomerlösung vorzugsweise hergestellt wird durch:
a. Mischen des mindestens einen hydrophoben Monomers und des mindestens einen hydrophilen Monomers in Wasser, um eine Zwischenlösung zu bilden; und
b. Zugabe der mindestens einen Aminosäure und des Vernetzers zur Zwischenlösung zwecks Bildung der Co-Monomerlösung,
wobei das hydrophobe Monomer und der Vernetzer verschiedene chemische Spezies sind.

10. Vorgang nach einem vorhergehenden Anspruch, ferner umfassend den Schritt des Hydratisierens des Copolymers nach der Polymerisation, wobei entweder das Copolymer vorzugsweise für mindestens 7 Tage nach der Hydratation gelagert wird, oder wobei das Copolymer so hydratisiert wird, dass das hydratisierte Copolymer mindestens 10 Gew.-% Wasser umfasst, bezogen auf das Gesamtgewicht des hydratisierten Copolymers.

11. Homogenes, isotropes, elektronisch aktives, hydrophiles Copolymer, das durch den Vorgangnach einem der Ansprüche 1-10 herstellbar ist.

12. Co-Monomerlösung nach Anspruch 1, umfassend mindestens ein hydrophobes Monomer, mindestens ein hydrophiles Monomer, Wasser, mindestens eine Aminosäure und mindestens einen Vernetzer.

13. Biokompatible medizinische Vorrichtung oder eine flexible biokompatible Nervenkontaktvorrichtung oder ein Cochlea-Implantat oder eine Herzschrittmacherelektrode, umfassend ein Copolymer nach Anspruch 11.

14. Biokompatible medizinische Vorrichtung, umfassend einen Superkondensator, wobei der Superkondensator zwei Elektroden und ein Copolymer nach Anspruch 11 umfasst, die dazwischen angeordnet sind, wobei die Vorrichtung vorzugsweise eine Nervenkontaktvorrichtung, ein Cochlea-Implantat oder ein Herzschrittmacher ist.

15. Biokompatibles Erfassungssystem, umfassend ein Copolymer nach Anspruch 11 mit einer in seiner gesamten Struktur verteilten chemischen Komponente, worin die chemische Komponente in der Lage ist, eine bestimmte Verbindung nachzuweisen.

## Revendications

1. Procédé de formation d'un co-polymère hydrophile actif réticulé électroniquement comprenant les étapes consistant à :
fournir une solution de monomère comprenant au moins un monomère hydrophobe, au moins un monomère hydrophile, de l'eau, au moins un acide aminé et au moins un réticulant ; et à
polymériser la solution de monomère ;
l'au moins un monomère hydrophile étant choisi parmi l'acide méthacrylique, le méthacrylate d'hydroxyéthyle, l'acrylate d'éthyle, la vinyl pyrrolidone, l'ester méthylique d'acide propénoïque, le phtalate de monométhacryloyloxyéthyle, le méthacrylate de sulphatoéthyle d'ammonium, le polyalcool vinylique ou une combinaison de ceux-ci ; et
l'au moins un monomère hydrophobe étant choisi parmi le méthacrylate de méthyle, le méthacrylate d'allyle, l'acrylonitrile, le méthacryloxypropyltris (triméthylsiloxy) silane, le 2,2,2-trifluoroéthyl méthacrylate, ou une combinaison de ceux-ci.

2. Procédé selon la revendication 1, dans lequel l'au moins un acide aminé est choisi parmi la phénylalanine, le tryptophane, l'histidine, l'acide éthylènediaminetétraacétique (EDTA) et la tyrosine, ou une combinaison de ceux-ci, de préférence l'au moins un acide aminé étant choisi parmi la phénylalanine et le tryptophane, ou une combinaison de ceux-ci.

3. Procédé selon l'une quelconque des revendications précédentes, dans lequel l'au moins un monomère hydrophile est choisi parmi la vinylpyrrolidone et le méthacrylate d'hydroxyéthyle, ou une combinaison de ceux-ci.

4. Procédé selon l'une quelconque des revendications précédentes, dans lequel l'au moins un monomère hydrophobe est choisi parmi l'acrylonitrile et le méthacrylate de méthyle, ou une combinaison de ceux-ci.

5. Procédé selon l'une quelconque des revendications précédentes, dans lequel l'agent de réticulation est le méthacrylate d'allyle ou le diméthacrylate d'éthylène glycol.

6. Procédé selon l'une quelconque des revendications précédentes, dans lequel le réticulant et le monomère hydrophobe sont à la fois le méthacrylate d'allyle.

7. Procédé selon l'une quelconque des revendications précédentes, dans lequel l'étape de polymérisation est réalisée par rayonnement thermique, UV ou gamma, de préférence l'étape de polymérisation étant réalisée par rayonnement UV ou gamma.

8. Procédé selon l'une quelconque des revendications précédentes, dans lequel la solution de co-monomère comprend en outre un initiateur de polymérisation, de préférence l'initiateur de polymérisation étant l'azobisisobutyronitrile ou la 2-hydroxy-2-méthylpriophénone.

9. Procédé selon l'une quelconque des revendications 1 à 5 ou 7 à 8, dans lequel la solution de co-monomère est obtenue en ajoutant l'acide aminé, sous la forme d'un solide, à au moins l'un des composants restants de la solution de co-monomère, de préférence la solution de co-monomère étant fournie par :
a. le mélange de l'au moins un monomère hydrophobe et de l'au moins un monomère hydrophile dans l'eau pour former une solution intermédiaire ; et
b. l'ajout de l'au moins un acide aminé et de l'agent de réticulation à la solution intermédiaire pour former la solution de co-monomère
le monomère hydrophobe et l'agent de réticulation étant des espèces chimiques différentes.

10. Procédé selon l'une quelconque des revendications précédentes, comprenant en outre l'étape d'hydratation du co-polymère après polymérisation, de préférence dans lequel soit le co-polymère est stocké pendant au moins 7 jours après l'hydratation, soit le co-polymère est hydraté de sorte que le co-polymère hydraté comprend au moins 10 % en poids d'eau, sur la base du poids total du co-polymère hydraté.

11. Co-polymère hydrophile isotrope électroniquement actif et homogène pouvant être obtenu par le procédé selon l'une quelconque des revendications 1 à 10.

12. Solution de co-monomères, selon la revendication 1, comprenant au moins un monomère hydrophobe, au moins un monomère hydrophile, de l'eau, au moins un acide aminé et au moins un réticulant.

13. Dispositif médical biocompatible, ou dispositif de contact nerveux biocompatible flexible, ou implant cochléaire, ou électrode de stimulateur cardiaque, comprenant un co-polymère selon la revendication 11.

14. Dispositif médical biocompatible comprenant un supercondensateur, dans lequel le supercondensateur comprend deux électrodes et un co-polymère selon la revendication 11 situés entre eux, le dispositif étant de préférence un dispositif de contact nerveux, un implant cochléaire ou un stimulateur cardiaque.

15. Système de détection biocompatible comprenant un co-polymère selon la revendication 11 avec un composant chimique dispersé dans toute sa structure, dans lequel ledit composant chimique est capable de détecter un composé particulier.
